# EUROPÄISCHE PATENTANMELDUNG

(11) **EP 1 475 110 A1**
(43) Veröffentlichungstag der Anmeldung: **10.11.2004**
(21) Anmeldenummer: 04011080.1
(22) Anmeldetag: 10.05.2004
(51) Int. Cl.: A61L 31/12, A61L 31/14, A61L 31/16

(54) **Stent zur kontrollierten Abgabe von Wirkstoffen**

(30) Priorität: 09.05.2003 DE 10320772; 09.05.2003 DE 10320773
(71) Anmelder: B. BRAUN MELSUNGEN AG, 34212 Melsungen (DE)
(72) Erfinder: Boxberger, Michael, 12103 Berlin (DE); Nagel, Stefan, 13503 Berlin (DE); Kühler, Michael, 10407 Berlin (DE); Schrör, Karsten, 50226 Frechen (DE)
(74) Vertreter: WALTHER, WALTHER & HINZ Patentanwälte

(57) **Zusammenfassung**

Gegenstand der Erfindung ist ein Stent zur Abgabe von unterschiedlichen an ihm angebrachten Wirkstoffen an einen menschlichen oder tierischen Körper während der Stent im Körper implantiert ist. Zur besseren medizinischen Applikation der Wirkstoffe wird vorgeschlagen, dass ein erster Wirkstoff als ein Sofortwirkstoff (D2) und ein zweiter Wirkstoff als ein Langzeitwirkstoff (D1) ausgebildet ist, wobei der Sofortwirkstoff (D2) in einem Schnellprozess und der Langzeitwirkstoff (D1) in einem Langzeitprozess an den Körper abgegeben wird.

## Beschreibung

Die vorliegende Erfindung betrifft ein Verfahren zur Abgabe von verschiedenen an einem Stent angebrachten Wirkstoffen an einen menschlichen oder tierischen Körper während der Stent im Körper implantiert ist, gemäß dem Oberbegriff des Anspruches 1 und einen Stent zur Implantation in einen Lumen eines menschlichen oder tierischen Körpers, gemäß den Oberbegriffen der Ansprüche 10 und 24.

Bei der interventionellen Therapie der koronaren Herzkrankheit tritt bei etwa 20 % - 40 % der Patienten bedingt durch die Implantation eines Stents in ein Blutgefäß innerhalb von 6 Monaten eine Restenose auf. Diese ist bedingt durch die Proliferation von Muskelzellen als biologische Antwort auf das Implantat. Zur Vorbeugung und Behandlung der Restenose wird der Stent mit einer antiproliferationale Substanz beschichtet, so dass der Wirkstoff unmittelbar an das betreffende Gewebe gelangt.

Aus der WO 01/87375 oder der EP 0 875 218 A 2 ist bekannt, an der dem Blutgefäß zugewandten Oberfläche des röhrenförmigen Stents Aussparungen vorzusehen, in welche ein erster Wirkstoff eingelagert werden kann, während der gesamte Stent mit einer einen zweiten Wirkstoff tragenden Schicht überzogen ist. Der zweite Wirkstoff kann dabei in ein Polymer eingebunden sein. Es versteht sich, dass die Art der Aussparung oder die Art des verwendeten Polymers nicht auf die in der WO 01/87375 oder EP 0 875 218 A 2 gewählten Ausführungsform beschränkt ist.

Sobald der Stent an der gewünschten Stelle implantiert ist, wird der zweite Wirkstoff an die den Stent umspülende Körperflüssigkeit, insbesondere an das Blut, und/oder an das den Stent umgebende Gewebe abgegeben. Nachdem sich diese Schicht aufgelöst hat, wird der erste Wirkstoff an den Körper abgegeben. In einer anderen Ausführungsform der WO 01/87375 sind die unterschiedlichen Wirkstoffe an verschiedenen Bereichen des Stens angebracht, so dass die beiden Wirkstoffe parallel abgegeben werden.

Aus der WO 98/56312 ist ein Stent bekannt, der mit zwei Polymerschichten überzogen ist, wobei in jeder Polymerschicht einer oder mehrere Wirkstoffe eingelagert sind. Dabei sind die Polymerschichten so eingestellt, dass die Wirkstoffe mit unterschiedlichen Abgaberaten freigesetzt werden. Gemäß der WO 98/56312 kann dabei, je nach Anwendungszweck, die Äußere Polymerschicht den Wirkstoff mit einer höheren Abgaberate und die Innere Polymerschicht den Wirkstoff mit einer niedrigeren Abgaberate freisetzen oder umgekehrt.

Der vorliegenden Erfindung liegt die Erkenntnis zugrunde, dass die vaskuläre Restenose oder ein anderes am Blutgefäß auftretendes Symptom am wirkungsvollsten behandelt werden könnte, wenn unmittelbar nach der Implantation eine therapeutisch sinnvolle Menge eines das Symptom bekämpfenden Wirkstoffes zur Verfügung stünde, um die aufkommenden Symptome gleich im Entstehen intensiv und wirkungsvoll medikamentös behandeln zu können und wenn darüber hinaus über einen längeren Zeitraum ein dem Symptom vorbeugendes Mittel zur Verfügung steht, um ein erneutes Ausbrechen des Symptoms zu verhindern.

Davon ausgehend liegt der vorliegenden Erfindung die Aufgabe zugrunde, einen Stent zu schaffen, der unmittelbar nach der Implantation in kurzer Zeit eine therapeutisch sinnvolle Menge eines ersten Wirkstoffes freisetzt und der außerdem über einen längeren Zeitraum einen zweiten Wirkstoff bereitstellt.

Als technische Lösung dieser Aufgabe wird erfindungsgemäß ein Verfahren mit den Merkmalen des Anspruches 1, ein Stent mit den Merkmalen des Anspruches 10 vorgeschlagen. Vorteilhafte Weiterbildungen des Verfahrens und des Stents sind den jeweiligen Unteransprüchen zu entnehmen.

Ein nach dieser technischen Lehre ausgebildeter Stent und ein nach dieser technischen Lehre ausgebildetes Verfahren zur Abgabe verschiedener an einem Stent angebrachter Wirkstoffe haben den Vorteil, dass unmittelbar nach der Implantation des Stentes in kürzester Zeit, vorzugsweise innerhalb von 18 Stunden, als sogenannter Sofortwirkstoff ein das Symptom bekämpfendes Mittel direkt an die kritische Stelle im Blutgefäß gelangt und das nach Bekämfpung der Symptome für einen längeren Zeitraum, vorzugsweise einige Wochen bis zu sechs Monaten, ein sogenannter Langzeitwirkstoff zur beschleunigten Heilung der Gefäßwand und/oder zur Vorbeugung gegen ein erneutes Auftreten des Symptoms zur Verfügung steht. Durch den zeitlich genau dosierten Einsatz des jeweiligen Wirkstoffes wird eine optimale Behandlung der Symptome erreicht. Auch durch die genaue Dosierung kann der Wirkstoff effektiv eingesetzt werden, so dass insgesamt weniger Wirkstoff verwendet wird, was über eine Kostenreduzierung, gleichzeitig auch eine Reduzierung etwaiger unerwünschter Nebenwirkungen zur Folge hat.

Beispielsweise zur Bekämpfung einer vaskulären Restenose hat sich als vorteilhaft erwiesen, innerhalb der ersten Stunden nach der Implantation des Stents etwa 20 µg des Sofortwirkstoffes Trapidil zusammen mit dem Stent an die kritische Stelle im Gewebe des Blutgefäßes zu bringen und gleichzeitig für einen längeren Zeitraum von bis zu sechs Monaten 200 µg eines Langzeitwirkstoffes, insbesondere eines Steroides, bereitzustellen. Dabei bekämpft das Trapidil die vaskuläre Restenose, während das Steroid die Gefäßheilung fördert und so einer Restenose vorbeugt.

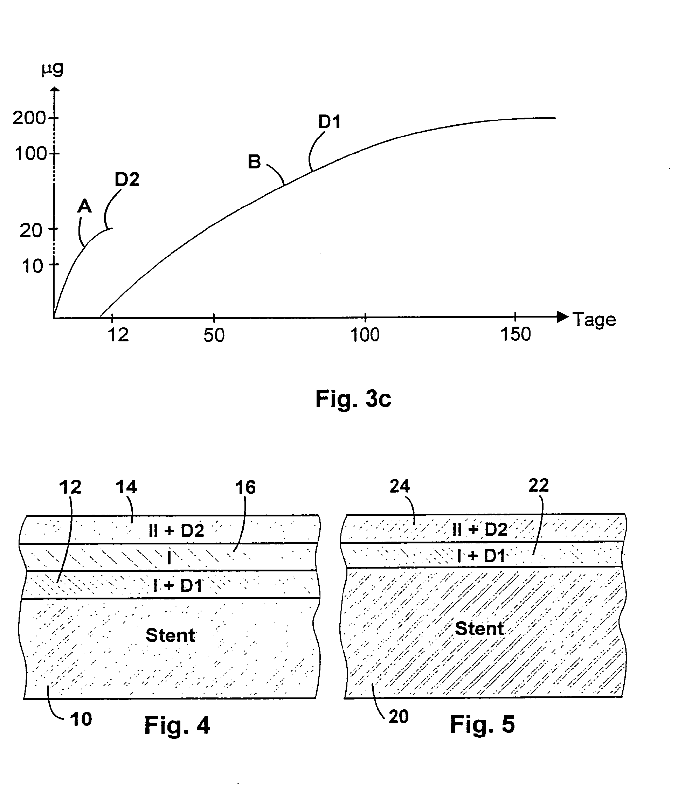

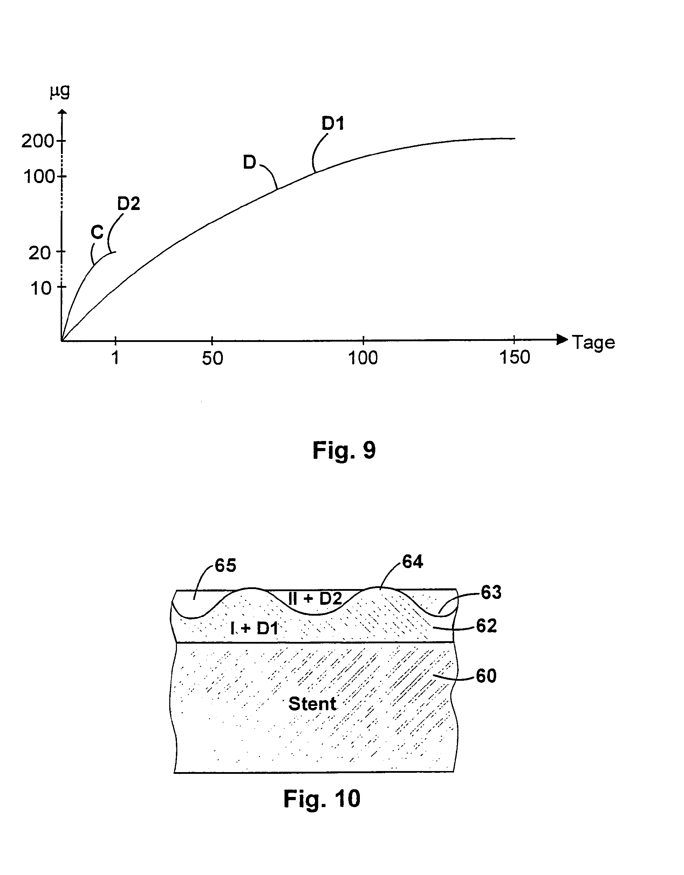

In einer bevorzugten Ausführungsform hat es sich als Vorteilhaft erwiesen, den Sofortwirkstoff und den Langzeitwirkstoff in unterschiedlichen Abgaberaten freizusetzen, um eine schnelle Bereitstellung der erforderlichen Menge des Sofortwirkstoffes und einer lang anhaltenden Bereitstellung des Langzeitwirkstoffes Rechnung zu tragen. Zur Erreichung dieser unterschiedlichen Abgabeprozesse sind die beiden Wirkstoffe in zwei unterschiedlichen, am Stent ausgebildeten Schichten eingelagert. Der für den Langzeitprozess einzusetzende Langzeitwirkstoff wird dabei in einer Basischicht gehalten, während der für den Schnellprozess einzusetzende Sofortwirkstoff in einer Wirkstoffschicht gehalten ist. Je nach Wirkstoff und Abgabeverlauf ist die Basisschicht unterhalb der Wirkstoffschicht angeordnet (siehe bspw. Figuren. 4 bis 7) oder beide Schichten sind abwechselnd nebeneinander angeordnet (siehe Fig. 10)

Dabei wird der Langzeitprozess insbesondere dadurch erreicht, dass der Langzeitwirkstoff in an sich bekannter Weise in Aussparungen in der Oberfläche des Stents eingelassen ist oder mittels einer für einen längeren Zeitraum an dem Stent anhaftenden Polymerschicht gehalten ist. Der Schnellprozess wird beispielsweise dadurch realisiert, dass auf der Basisschicht eine zweite, spezielle Wirkstoffschicht vorgesehen ist, in der der Sofortwirkstoff gehalten ist. Dabei wird diese Wirkstoffschicht so gestaltet, dass der Sofortwirkstoff vergleichsweise schnell freigesetzt wird. Es hat sich als vorteilhaft erwiesen, den Sofortwirkstoff im Schnellprozess zwischen sechs und zwanzig Mal schneller abzugeben, als den Langzeitwirkstoff des Langzeitprozesses, wobei der Schnellprozess nach einigen Stunden abgeschlossen ist, wohingegen der Langzeitprozess mehrere Wochen oder Monate andauert.

In einer bevorzugten Ausführungsform besteht die den Sofortwirkstoff haltende Wirkstoffschicht wahlweise aus einem längere Zeit am Stent verbleibenden oder aus einem sich schnell abbauenden Trägermaterial. Das lange am Stent verbleibende Trägermaterial bildet eine gewisse hemmende Schicht und verlangsamt die Abgabe des unter dem Trägermaterial befindlichen Langzeitwirkstoffes. Somit ist die Anordnung eines solchen lange anhaftenden Trägermaterials ein Mittel zur Steuerung der Wirkstoffabgabe.

Bei einem sofort nach der Implantation des Stents im Körper abzubauendem Trägermaterial verschwindet die Wirkstoffschicht nach kurzer Zeit, beispielsweise nach 12 Stunden bis 36 Stunden, vorzugsweise 18 Stunden. Sodann hat der unter der Wirkstoffschicht liegende Stent direkten Kontakt mit dem umgebenden Gewebe bzw. der Körperflüssigkeit, mit der Folge, dass der Langzeitwirkstoff nun direkt, also viel schneller abgegeben wird. Es versteht sich, dass die Anbringung des Wirkstoffes am Stent in der aus dem Stand der Technik bekannten Art und Weise erfolgt.

Die Auswahl einer geeigneten Basisschicht und einer geeigneten Wirkstoffschicht und die Auswahl der geeigneten Wirkstoffe erfolgt in der aus dem Stand der Technik bekannten Art und Weise, wobei die Auswahl von den im Einzelfall gegebenen Umständen abhängt. Beispielhafte Ausführungsformen hierfür sind in der Figurenbeschreibung gegeben.

In einer weiteren, bevorzugten Ausführungsform hat es sich als vorteilhaft erwiesen, sowohl den Schnell-, als auch den Langzeitprozess unmittelbar nach Implantation des Stents einsetzen zu lassen. Werden im Schnell- und im Langzeitprozess dieselben Wirkstoffe eingesetzt, so wird hierdurch die Menge des schnell freigesetzten Wirkstoffes noch weiter erhöht. Werden im Schnellund im Langzeitprozess unterschiedliche Wirkstoffe eingesetzt, so wird hierdurch erreicht, dass der der Restenose vorbeugende Langzeitwirkstoff sehr früh an das Gewebe gelangt und seine prophylaktische Wirkung zeitgleich mit der medikamentösen Behandlung der Restenose einsetzt, so dass ein Befall des noch gesunden Gewebes mit Restenose unterdrückt wird. Erreicht wird dies in einer vorteilhaften Ausführungsform dadurch, dass die als Polymerschicht ausgebildete Basisschicht an unterschiedlichen Stellen eine unterschiedliche Schichtdicke aufweist, so dass die Polymerschicht Täler und Berge ausbildet, wobei in den Tälern die den Sofortwirkstoff haltende Wirkstoffschicht untergebracht ist. Bei diesem Aufbau des Stents kommt das um den Stent herum liegende Gewebe sowohl mit der Basisschicht (im Bereich der Berge), als auch mit der Wirkstoffschicht (im Bereich der Täler) in Kontakt, so dass sowohl der Schnell-, als auch der Langzeitprozess unmittelbar nach Implantation des Stents in Gang gesetzt werden.

Als weitere technische Lösung dieser Aufgabe wird erfindungsgemäß ein Stent mit den Merkmalen des Anspruches 24 vorgeschlagen. Bei diesem Stent wird ein einziger Wirkstoff D sowohl über den Schnell-, als auch über den Langzeitprozess abgegeben. Dies hat den Vorteil, dass unmittelbar nach der Implantation des Stentes eine nennenswerte Menge des Wirkstoffes an die kritische Stelle im Gewebe des Blutgefäßes gelangt, um der vaskulären Restenose oder einem anderen Symptom bereits im frühen Stadium entgegenzuwirken und eine schnelle Heilung herbei zu führen. Dabei hat es sich als vorteilhaft erwiesen, einen für einen Schnellprozess geeigneten Wirkstoff zu verwenden.

Besonders Vorteilhaft ist dabei die Ausbildung der einzelnen Schichten mit unterschiedlichen Schichtdicken. Hierdurch kann sowohl der Schnellprozess mit einer hohen Abgaberate, als auch der Langzeitprozess mit einer niedrigen Abgaberate gleichzeitig einsetzen und nach Beendigung des Schnellprozesses wird dann nur noch der Langzeitprozess fortgeführt.

Weitere Vorteile des erfindungsgemäßen Verfahrens und des erfindungsgemäßen Stents ergeben sich aus der beigefügten Zeichnung und den nachfolgend beschriebenen Ausführungsformen. Ebenso können die vorstehend genannten und die noch weiter ausgeführten Merkmale erfindungsgemäß und jeweils einzeln oder in beliebigen Kombinationen miteinander verwendet werden. Die erwähnten Ausführungsformen sind nicht als abschließende Auszählung zu verstehen, sondern haben vielmehr beispielhaften Charakter.
Es zeigen:
- Fig. 1: eine perspektivische Ansicht einer ersten Ausführungsform eines erfindungsgemäßen Stentes in aufgeweitetem Zustand;
- Fig. 2: eine Ausschnittsvergrößerung des Stents gemäß Fig. 1, vergrößert entlang Linie II;
- Fig. 3 a -c: schematische Diagramme mit dem erfindungsgemäßen Verlauf der Abgabe der Wirkstoffe;
- Fig. 4 - 7: schematische Darstellungen weiterer Ausführungsformen erfindungsgemäßer Stents;
- Fig. 8: ein schematisches Diagramm mit einem weiteren, erfindungsgemäßen Verlauf der Abgabe der Wirkstoffe;
- Fig. 9: einen alternativen Verlauf der Abgabe der Wirkstoffe;
- Fig. 10: eine sechste Ausführungsform eines erfindungsgemäßen Stents;
- Fig. 11: eine siebte Ausführungsform eines erfindungsgemäßen Stents.

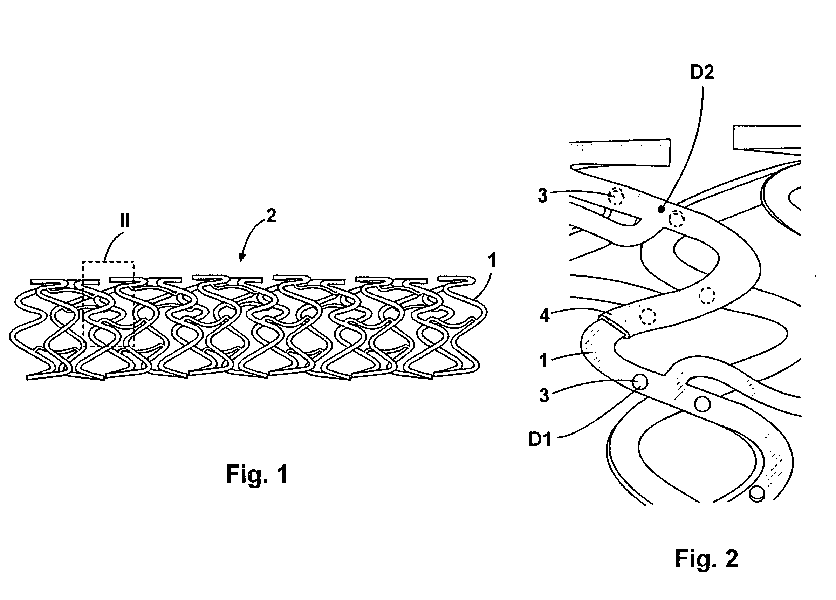

Die Figuren 1 und 2 zeigen beispielhaft einen aus einer Vielzahl von Streben 1 zusammengesetzten Stent 2 in expandiertem Zustand. In dieser ersten Ausführungsform eines erfindungsgemäßen Stents sind auf der dem Blutgefäß zugewandten Seite der Strebe 1 eine Anzahl von sacklochartigen Aussparungen 3 eingelassen, die ein Depot zur Einlagerung eines Langzeitwirkstoffes D1 bilden. Die in der Oberfläche der Strebe 1 angeordneten Aussparungen 3 werden auch als Basisschicht bezeichnet. Die Streben 1, respektive die Basisschicht, sind mit einer Wirkstoffschicht 4 aus einem nur sehr langsam im Körper abbaubaren Polymer überzogen, in der ein Sofortwirkstoff D2 gehalten ist. Dieses Polymer ist beispielsweise Polymethylacrylat oder ein Derivat hiervon. In anderen, hier nicht näher dargestellten Ausführungsformen kann das Polymer alternativ aus PC, PA, PET, PE, PVA, PVP, PUR, PAN, PEO, PESU, Polyacryl, Polycarboxylsäure, einem Derivat eines der vorgenannten Materialien oder einer Kombination einiger der genannten Materialien gebildet sein.

Die Wirkstoffschicht 4 aus Polymethylacrylat ist wirkstoffdurchlässig und baut sich im Körper nur sehr langsam ab. In dieser Ausführungsform werden mehrere Jahre vergehen, bis die Wirkstoffschicht 4 vollständig abgebaut ist. Aufgrund der Wirkstoffdurchlässigkeit des Polymers kann der unter der Wirkstoffschicht 4 in den Aussparungen 3 des Depots befindliche Langzeitwirkstoff D1 durch das Polymethylacrylat hindurch wandern und in den Körper gelangen.

Zur Vorbeugung und Bekämpfung einer vaskulären Restenose wird in dieser ersten Ausführungsform als Sofortwirkstoff D2 Trapidil eingesetzt. Das schnell wirkende Trapidil unterbricht die mitogene Signalübertragung zu den Muskelzellen und verhindert somit ein Ausbreitung der Restenose. Dieser Sofortwirkstoff D2 ist gleichmäßig in der Wirkstoffschicht 4 eingelagert und wird sowohl an der dem Blutgefäß zugewandten Seite der Strebe 1, als auch an anderen Seiten der Strebe 1 freigesetzt, da die Wirkstoffschicht 4 die Strebe 1 vollständig umgibt. Dabei gelangt der Sofortwirkstoff D2 in das Gewebe des Blutgefäßes und in die den Stent 2 umgebenden Körperflüssigkeiten, vornehmlich in das Blut des Patienten.

Ergänzend zum schnell wirkenden Sofortwirkstoff D2 zur Bekämfpung akuter Krankheitssymptome ist in der Basisschicht ein zweiter, über einen längeren Zeitraum einzusetzender Langzeitwirkstoff D1 vorgesehen. Dieser Langzeitwirkstoff D1 soll die Gefäßheilung und/oder die Bildung einer Endothelschicht unterstützen. In der vorliegenden Ausführungsform wird als Langzeitwirkstoff D1 ein Steroid eingesetzt.

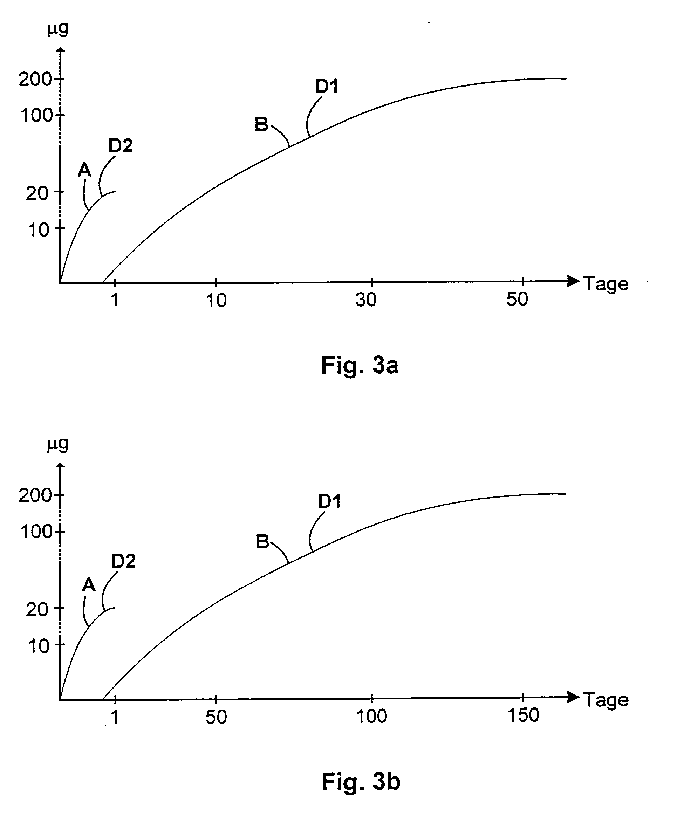

Nachdem der Stent 2 in an sich bekannter Weise im Lumen implantiert ist, kommt die äußere Wirkstoffschicht 4 in vollem Umfang mit den Körperflüssigkeiten und/oder mit dem Gewebe in Kontakt, wobei der in der Wirkstoffschicht 4 befindliche Sofortwirkstoff D2 im Rahmen eines Schnellprozesses freigesetzt wird. Bei diesem Schnellprozess wird in kurzer Zeit eine große Menge des Trapidils D2 aus der Wirkstoffschicht 4 an den Körper abgegeben, wobei die Wirkstoffschicht 4 aus Polymethylacrylat in an sich bekannter Weise so eingestellt ist, dass sich während des Schnellprozesses eine Abgaberate ergibt, in der in einem Zeitraum von etwa 18 Stunden bis zu 50 µg, vorzugsweise 20µg, des Sofortwirkstoffes D2 freigesetzt werden. In dieser Ausführungsform erfolgt die Abgabe nicht linear, sondern zunächst ist die Abgaberate höher und wird mit der Zeit niedriger, wie der Kurve A im Diagramm gemäß Fig. 3 a zu entnehmen ist. Der Grund hierfür liegt darin, dass zunächst die nahe der Außenfläche eingelagerten Wirkstoffmoleküle freigesetzt werden, die einen direkten Kontakt zum Gewebe oder zur Körperflüssigkeit haben. Anschließend müssen die im Inneren der Wirkstoffschicht 4 gehaltenen Wirkstoffmoleküle an die Außenfläche nachrücken, bevor diese an den Körper abgegeben werden können, was naturgemäß etwas Zeit in Anspruch nimmt, so dass die Abgaberate nun unwesentlich sinkt.

Unabhängig vom Schnellprozess wird das im Depot als Langzeitwirkstoff D1 eingelagerte Steroid in einem Langzeitprozess freigesetzt. Hierbei wandert der im Depot befindliche Langzeitwirkstoff D1 über die Kontaktfläche zwischen Aussparung 3 und Wirkstoffschicht 4 zunächst in die Wirkstoffschicht 4 und wird anschließend an den Körper abgegeben. Dabei rückt weniger Wirkstoff D1 vom Depot in die Wirkstoffschicht 4 nach, als von dieser an die Umgebung abgegeben werden könnte. Folglich kann durch die Anzahl und Größe der Aussparungen, sowie durch eine Gestaltung der Polymereigenschaften, insbesondere des Vernetzungsgrades, die Abgaberate während des Langzeitprozesses eingestellt werden. Hierdurch wird auch erreicht, dass die Abgaberate im Langzeitprozess sehr viel niedriger liegt als die Abgaberate im Schnellprozess. Tatsächlich werden beim Langzeitprozess über einen Zeitraum von ungefähr sechs bis sieben Wochen bis zu 500 µg, vorzugsweise etwa 200 µg abgegeben, wie Kurve B des Diagramms gemäß Fig. 3 a zu entnehmen ist, so dass die Abgaberate des in der Wirkstoffschicht 4 eingelagerten Sofortwirkstoffes D2 während des Schnellprozesses circa 6 Mal höher als die Abgaberate des in der Basisschicht 3 eingelagerten Langzeitwirkstoffes D1 während des Langzeitprozesses ist.

Die kleine Kontaktfläche bewirkt auch, dass während des Schnellprozesses nur eine vernachlässigbar geringe Menge des Langzeitwirkstoffes D1 von den Depots bis in den Körper gelangt. Ein weiterer Grund für das vernachlässigbare Nachrücken des Langzeitwirkstoffes D1 aus dem Depot während des Schnellprozesses liegt darin, dass die im Polymer der Wirkstoffschicht 4 eingebundenen Wirkstoffmoleküle viele der freien Plätze belegen und somit kein oder nur wenig Platz für die aus dem Depot nachrückenden Wirkstoffmoleküle zur Verfügung steht.

Mit Beendigung des Schnellprozesses, im vorliegenden Fall ungefähr nach Ablauf der ersten achtzehn Stunden, das heißt wenn zumindest ein Grossteil des Sofortwirkstoffes D2 der Wirkstoffschicht 4 freigesetzt ist, setzt der Langzeitprozess ein, während dessen der Langzeitwirkstoff D1 aus den Depots der Basisschicht 3 in die Wirkstoffschicht 4 nachrückt und über diese in den Körper gelangt. In dieser Ausführungsform ist der Übergang vom Schnellprozess zum Langzeitprozess fließend, da der Langzeitwirkstoff D1 kontinuierlich in die Wirkstoffschicht 4 nachrückt.

Die Darstellungen in Fig. 3 a, und in den weiter unten noch beschriebenen Figuren 3 b und 3 c, geben den Verlauf der Wirkstoffabgabe lediglich schematisch wieder und sind nicht maßstabsgetreu. Die konkreten Kennlinien für Stents gemäß den hier aufgezählten Ausführungsformen können hiervon Abweichen, verlaufen jedoch nach den oben beschriebenen Grundsätzen. Es versteht sich, dass die konkrete Abgabegeschwindigkeit der Wirkstoffe D1 und D2 und die konkrete Abgabemenge der Wirkstoffe D1 und D2 vom Krankheitsbild abhängt, wobei der behandelnde Arzt diese Werte festlegt.

Es versteht sich ferner, dass alternativ oder zur Bekämpfung anderer Symptome oder Krankheitsbilder in anderen, hier nicht näher dargestellten Ausführungsformen, auch andere Sofortwirkstoffe oder Kombinationen mehrerer Wirkstoffe eingesetzt werden können. Dies können beispielsweise Integrin Antagonisten (z. B. Zyklopeptide), Thrombozytenaggregationshemmer, Heparin, Hirudin, Statine, Zytostatika, Immunsuppressiva, Hormone, ein Derivat eines der vorgenannten Wirkstoffe oder eine Kombination mehrerer der vorgenannten Wirkstoffe sein. Auch kann der Schnell- oder der Langzeitprozess einen anderen zeitlichen Verlauf nehmen.

So kann der Langzeitprozess beispielsweise in einer anderen Ausführungsform durch Verwendung eines anderen Polymers und/oder durch Verkleinern der mit der Wirkstoffschicht 4 in Kontakt stehenden Oberfläche der Aussparung 3 auf mehrere, beispielsweise fünf Monate, erstreckt werden, wie im Diagramm gemäß Fig. 3 b dargestellt ist.

In Fig. 3 c ist ein weiterer Verlauf der Wirkstoffabgabe einer weiteren Ausführungsform dargestellt, bei der das Polymer der Wirkstoffschicht so eingestellt ist, dass sich der Schnellprozess über circa 10 bis 12 Tage erstreckt, während sich der Langzeitprozess über circa fünf Monate erstreckt.

Bei den vorgenannten Ausführungsformen werden im Schnellprozess zwischen 5 µg und 50 µg, vorzugsweise 20 µg, des Sofortwirkstoffes D2 Trapidil freigesetzt, während im Langzeitprozess zwischen 50 µg und 500 µg, vorzugsweise 200 µg des Langzeitwirkstoffes D1 Steroid freigesetzt werden.

In einer weiteren, hier nicht dargestellten Ausführungsform werden etwa 10 µg des Sofortwirkstoff D2 im Schnellprozess in weniger als 5 Stunden abgegeben, während vom Steroid im Langzeitprozess über 6 Monate etwa 200 µg freigesetzt werden.

Die Figuren 4 bis 7 zeigen alternative Ausführungsformen des erfindungsgemäßen Stents, deren Wirkstoffabgabe nach dem erfindungsgemäßen Verfahren erfolgt. Dabei ist der Stent jeweils nur schematisch angedeutet.

In der zweiten Ausführungsform gemäß Fig. 4, ist auf einer Strebe 10 eine Basisschicht 12 aus einem Polymer I aufgetragen, die den Langzeitwirkstoff D1 für den Langzeitprozess hält. Auf dieser Basisschicht 12 ist eine wirkstoffdurchlässige Schutzschicht 16 aus einem Polymer I aufgetragen, wobei in dieser Ausführungsform für die Basisschicht 12 und die Schutzschicht 16 dasselbe Polymer I eingesetzt ist. Auf der Schutzschicht 16 ist eine Wirkstoffschicht 14 angeordnet, die ein leicht im Körper abbaubares Trägermaterial II und einen vom Trägermaterial gehaltenen Sofortwirkstoff D2 für den Schnellprozess umfasst.

In der dritten Ausführungsform gemäß Fig. 5 ist auf einer Strebe 20, ebenso wie in der zweiten Ausführungsform, eine Basisschicht 22 aus einem Polymer I aufgetragen, die den Langzeitwirkstoff D1 für den Langzeitprozess hält. Eine Schutzschicht ist hier nicht vorgesehen, so dass in dieser Ausführungsform eine den Sofortwirkstoff D2 haltende Wirkstoffschicht 24 direkt auf der Basisschicht 22 aufliegt. Auch hier umfasst die Wirkstoffschicht 24 ein leicht im Körper abbaubares Trägermaterial II, in dem der Sofortwirkstoff D2 gehalten ist.

In der zweiten Ausführungsform gemäß Fig. 4 ist das leicht abbaubare Trägermaterial II eine Emulsion, vorzugsweise Lebertran (COD liver oil), während das flüchtige Trägermaterial II in der dritten Ausführungsform ein schnell abbaubares Polymer, insbesondere ein Polyphosphazen, ein Polyanhydrid, ein Polydioxan, ein Polyacetal, ein Polyoxalat ein Polyester oder ein Derivat eines dieser Materialien ist. In beiden Fällen wird das Trägermaterial II abgebaut und in das Gewebe, bzw. in die Körperflüssigkeit transportiert, so dass der Sofortwirkstoff D2 freigesetzt wird und ebenfalls in den Körper gelangt.

Die Basisschicht 12, 22 und die Schutzschicht 16 sind aus einem dauerhaft anhaftenden Polymer I gebildet, welches auch bei Kontakt mit Gewebe und/oder Körperflüssigkeiten auf dem Stent verbleibt oder nur sehr langsam abgebaut wird. Ein solches Polymer I ist beispielsweise Polymethylacrylat oder ein Derivat hiervon. In anderen, hier nicht dargestellten Ausführungsformen, kann diese Basisschicht 12, 22 und/oder die Schutzschicht 16 aus PC, PA, PET, PE, PVA, PVP, PUR, PAN, PEO, PESU, Polyacryl, Polycarboxylsäure, ein Derivat eines der vorgenannten Materialien oder einer Kombination einiger der vorgenannten Materialien sein.

Als Langzeitwirkstoff D1 wird vorzugsweise ein Steroid eingesetzt. Es ist aber auch möglich, ein Anti-Coagulant, ein PAR Inhibitor, ein Derivat eines der vorgenannten Wirkstoffe oder eine Kombination einiger der vorgenannten Wirkstoffe einzusetzen.

Als Sofortwirkstoff D2 wird vorzugsweise Trapidil eingesetzt. Es ist aber auch möglich ein Anti-Inflammatorium, z. B. Dexamethasone, Corticosteroid, M-Prednisolone, Interferon Y - 1 b, Leflunomide, Sirolismus, Tacrolismus, Mofatil, Cyclosporine,Transilast, etc., ein Anti-Proliferativum, wie beispielsweise Zytostatika oder Integrin Antagonisten, z. B. Rapamyzin, QP-2, Taxol, Vincristine, Angiopeptin, Statine, Mitimycine, Antinomycine, Methothredxate, etc., einen Thrombozyten Aggregationshemmer, z. B. Hirudin, Heparin, PAR Inhibitoren, etc., einen Migration Inhibitor, z. B. Batimastat, Prolylhydrooxylase-Inhibitoren, Halofuginone, C-Porteinase-Inhibitoren, ein Immunsuppressiva, ein Derivat eines der vorgenannten Wirkstoffe oder eine Kombination einiger der vorgenannten Wirkstoffe einzusetzen.

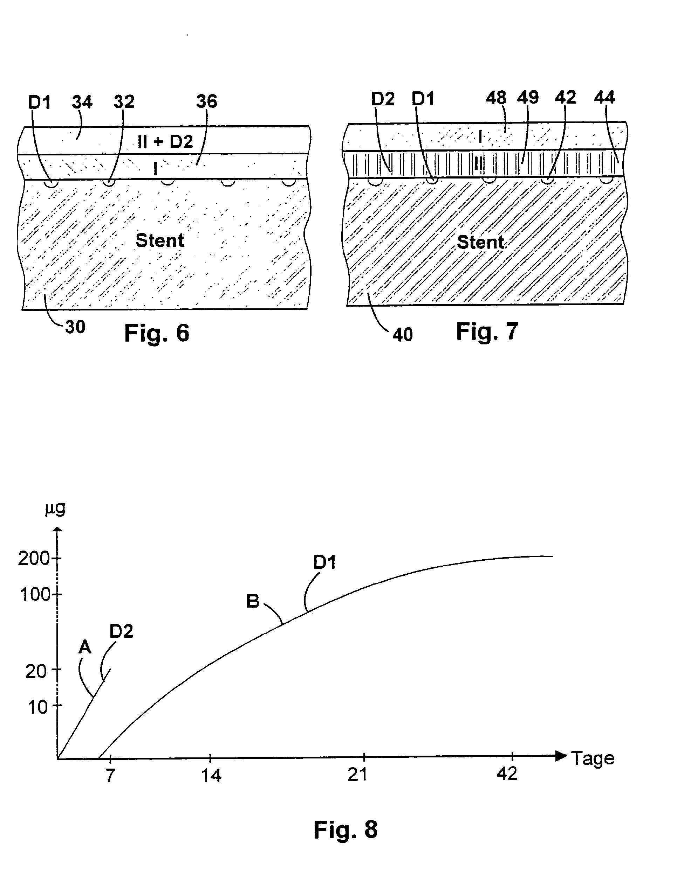

Wie bei der ersten Ausführungsform, ist der für den Langzeitprozess bestimmte Langzeitwirkstoff D1 in der vierten und fünften Ausführungsform gemäß den Figuren 6 und 7 ebenfalls in einer in der Strebe 30, 40 eingelassenen Basisschicht 32, 42 eingelagert, wobei der Langzeitwirkstoff D1 in entsprechenden Aussparungen in der Oberfläche der Strebe 30, 40 gehalten ist.

Im Gegensatz zur ersten Ausführungsform ist in der vierten Ausführungsform gemäß Fig. 6 auf der Strebe 30 eine Schutzschicht 36 vorgesehen. Diese Schutzschicht 36 ist analog zur Schutzschicht 16 gemäß der zweiten Ausführungsform ebenfalls aus einem der zuvor genannten Polymere gebildet. Auf dieser Schutzschicht 36 ruht dann eine Wirkstoffschicht 34 umfassend ein flüchtiges Trägermaterial II , in diesem Fall eine Emulsion mit dem für den Schnellprozess vorgesehenen Sofortwirkstoff D2, in diesem Fall ein Angiopeptin.

In der fünften Ausführungsform gemäß Fig. 7 ist auf der Basisschicht 42 direkt, das heißt ohne eine zwischengeschaltete Schutzschicht, eine den Sofortwirkstoff D2 für den Schnellprozess tragende Wirkstoffschicht 44 vorgesehen. Diese Wirkstoffschicht 44 umfasst ein nicht flüchtiges, keramisches Trägermaterial, vorzugsweise Aluminiumoxid, in welches der Sofortwirkstoff D2 eingelagert ist. Dabei ist die Wirkstoffschicht 44 von einer Vielzahl von senkrecht zur Strebe 40 angeordneten Kanälen 49 durchzogen, in denen der Sofortwirkstoff D2 eingelagert ist. Auf dieser Wirkstoffschicht 44 ist dann eine Schutzschicht 48 aus einem zuvor genannten Polymer angebracht, mit der die Freigabe des Sofortwirkstoffes D2 aus der Wirkstoffschicht 44 kontrollierbar ist.

Beispielsweise beim Einsatz eines flüchtigen Trägermaterials in der Wirkstoffschicht 34 gemäß der vierten Ausführungsform gemäß Fig. 6 verläuft die Wirkstoffabgabe im Schnellprozess linear, wie in Kurve 1 im Diagramm gemäß Fig. 8 dargestellt ist. Der Grund hierfür liegt darin, dass sich die Wirkstoffschicht 34 sukzessive auflöst und so über die Zeit betrachtet stets gleiche Bedingungen für die Freigabe des Sofortwirkstoffes D2 vorherrschen.

Das Diagramm gemäß Fig. 9 zeigt eine weitere Ausführungsform des erfindungsgemäßen Verfahrens und in Fig. 10 ist schematisch eine sechste Ausführungsformen eines erfindungsgemäßen Stents dargestellt, mit dem das Verfahren gemäß Fig. 9 ausführbar ist.

Im Diagramm gemäß Fig. 9 stellt die Kennlinie C den Schnellprozess dar, bei dem innerhalb von einem Tag etwa 20 µg des Sofortwirkstoffes D2 abgegeben werden. Zusammen mit dem Schnellprozess beginnt auch der Langzeitprozess, bei dem in einem Zeitraum von circa 150 Tagen insgesamt ca. 200 µg des Langzeitwirkstoffes D1 abgegeben werden, wie Kennlinie D darstellt. Es versteht sich, dass diese Kennlinien C, D die Abgabe des Wirkstoffes nur schematisch wiedergeben. Die konkreten Kennlinien für Stents gemäß den nachfolgenden Ausführungsformen können hiervon Abweichen, verlaufen jedoch nach den oben beschriebenen Grundsätzen.

Die in Fig. 10 dargestellte sechste Ausführungsform umfasst ebenfalls eine Strebe 60, auf dessen Oberfläche ein im Langzeitprozess abzugebender Langzeitwirkstoff D1 mittels einer Basisschicht 62 gehalten ist. Diese Basisschicht 62 ist analog zu den Basisschichten 12, 22 gemäß der zweiten und dritten Ausführungsformen auch aus einem sehr langsam abbauenden Polymer gebildet. Im Gegensatz zu den zuvor beschriebenen Ausführungsformen weist diese Basisschicht 62 lokal unterschiedliche Schichtdicken auf, so dass sich auf der Oberfläche des Stents Täler 63 und Berge 64 ausbilden. In diese Täler 63 ist dann die den Sofortwirkstoff D2 für den Schnellprozess tragende Wirkstoffschicht 65 eingebracht. Diese Wirkstoffschicht 65 kann ebenfalls aus einem der oben beschriebenen Trägermaterialen gebildet sein. Dabei wird die Wirkstoffschicht 65 derart in die Täler 63 eingebracht, dass die Berge 64 noch aus der Wirkstoffschicht 65 herausragen, so dass das Gewebe und/oder die Körperflüssigkeit gleichzeitig an der Wirkstoffschicht 65 und an der Basisschicht 62 anliegen, so dass der Sofortwirkstoff D2 aus der Wirkstoffschicht 65 gleichzeitig mit dem Langzeitwirkstoff D1 aus der Basisschicht 62 austreten kann. Hierdurch wird der Schnellprozess und der Langzeitprozess gleichzeitig in Gang gesetzt.

Es versteht sich, dass die hier erwähnten Materialien für die Basisschicht, die Schutzschicht und das Trägermaterial in anderen, hier nicht dargestellten Ausführungsformen, auch in beliebigen anderen Kombinationen eingesetzt werden können.

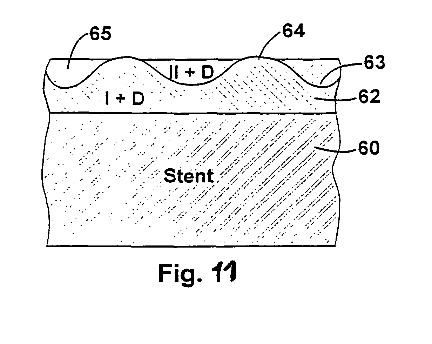

Die in Fig. 11 dargestellte siebte Ausführungsform unterscheidet sich von der in Fig 10 dargestellten sechsten Ausführungsform dadurch, dass hier lediglich ein einziger Wirkstoff D eingesetzt wird. Dieser Wirkstoff D wird gleichzeitig über einen gewissen Teil des Stents, nämlich über die Berge 64, im Langzeitprozess und über einen anderen Teil des Stents, nämlich im Bereich der Täler 63, im Sofortprozess abgegeben, so dass zu Beginn der Implantation des Stents eine vergleichsweise hohe Dosierung des Wirkstoffes D zur Verfügung steht, während der Wirkstoff D nach Beendigung des Sofortprozesses weiterhin im Langzeitprozess abgegeben wird, so dass der Wirkstoff D auch über einen langen Zeitraum zur Verfügung steht.

## Patentansprüche

1. Verfahren zur Abgabe von verschiedenen an einem Stent angebrachten Wirkstoffen an einen menschlichen oder tierischen Körper während der Stent im Körper implantiert ist,
**dadurch gekennzeichnet,**
**dass** ein erster Wirkstoff als Sofortwirkstoff (D2) und ein zweiter Wirkstoff als Langzeitwirkstoff (D1) ausgebildet ist, wobei der Sofortwirkstoff (D2) in einem Schnellprozess und der Langzeitwirkstoff (D1) in einem Langzeitprozess an den Körper abgegeben wird.

2. Verfahren nach Anspruch 1,
**dadurch gekennzeichnet,**
**dass** der Sofortwirkstoff (D2) ein Anti-Inflammatorium, z. B. Dexamethasone, Corticosteroid, M-Prednisolone, Interferon γ - 1b, Leflunomide, Sirolismus, Tacrolismus, Mofatil, Cyclosporine,Transilast, etc., ein Anti-Proliferativum, wie beispielsweise Zytostatika oder Integrin Antagonisten, z. B. Zyklopeptide, Rapamyzin, Trapidil, QP-2, Taxol, Vincristine, Angiopeptin, Statine, Mitimycine, Antinomycine, Methothredxate, einen Thrombozyten Aggregationshemmer, z. B. Hirudin, Heparin, einen Migration Inhibitor, z. B. Batimastat, Prolylhydrooxylase-Inhibitoren, Halofuginone, C-Porteinase-Inhibitoren, ein Immunsuppressiva, Hormone, ein Derivat eines der vorgenannten Wirkstoffe oder eine Kombination einiger der vorgenannten Wirkstoffe enthält.

3. Verfahren nach einem der vorangehenden Ansprüche,
**dadurch gekennzeichnet,**
**dass** der Langzeitwirkstoff (D1) ein Steroid, ein Anti-Coagulant, ein PAR Inhibitor, ein Derivat eines der vorgenannten Wirkstoffe oder eine Kombination einiger der vorgenannten Wirkstoffe enthält.

4. Verfahren nach einem der vorangehenden Ansprüche,
**dadurch gekennzeichnet,**
**dass** der Sofortwirkstoff (D2) im Schnellprozess mit einer höheren Abgaberate freigesetzt wird, als der Langzeitwirkstoff (D1) im Langzeitprozess.

5. Verfahren nach einem der vorangehenden Ansprüche,
**dadurch gekennzeichnet,**
**dass** der Langzeitprozess einsetzt, sobald oder kurz bevor der Schnellprozess abgeschlossen ist.

6. Verfahren nach einem der Ansprüche 1 bis 4,
d**adurch gekennzeichnet,**
dass sowohl der Schnell-, als auch der Langzeitprozess unmittelbar nach der Implantation des Stents einsetzen.

7. Verfahren nach einem der vorangehenden Ansprüche,
**dadurch gekennzeichnet,**
**dass** die höhere Abgaberate im Schnellprozess etwa 3 Mal bis 30 Mal, vorzugsweise 6 Mal bis 20 Mal höher als die niedrigere Abgaberate im Langzeitprozess ist.

8. Verfahren nach einem der vorangehenden Ansprüche,
**dadurch gekennzeichnet,**
**dass** die Abgaberate während des Schnellprozesses zwischen 3 µg und 50 µg, vorzugsweise zwischen 5 µg und 20 µg in einem Zeitraum von 2 Stunden bis 14 Tagen, vorzugsweise 5 Stunden bis 10 Tagen beträgt.

9. Verfahren nach einem der vorangehenden Ansprüche,
**dadurch gekennzeichnet,**
**dass** die Abgaberate während des Langzeitprozesses zwischen 20 µg und 500 µg, vorzugsweise zwischen 25 µg bis 200 µg in einem Zeitraum von 0,5 Monaten bis 10 Monaten, vorzugsweise 6 Wochen bis 6 Monate, beträgt.

10. Stent zur Implantation in einen Lumen eines menschlichen oder tierischen Körpers, mit einem in einer Basisschicht (12, 22, 32, 42, 62) am Stent (2, 10, 20, 30, 40, 60) lösbar gehaltenen ersten Wirkstoff (D1) und mit einem in einer zusätzlichen Wirkstoffschicht (4,14, 24, 34, 44, 65) am Stent (2, 10, 20, 30, 40, 60) lösbar gehaltenen zweiten Wirkstoff (D2),
**dadurch gekennzeichnet,**
**dass** der zweite Wirkstoff (D2) als Sofortwirkstoff ausgebildet ist, während der erste Wirkstoff (D1) als Langzeitwirkstoff ausgebildet ist, wobei der in der Basisschicht (12, 22, 32, 42, 62) eingelagerte Langzeitwirkstoff über einen längeren Zeitraum an den Körper abgegeben wird, als der in der Wirkstoffschicht (4, 14, 24, 34, 44, 65) gehaltene Sofortwirkstoff.

11. Stent nach Anspruch 10,
**dadurch gekennzeichnet,**
**dass** die Abgaberate der Wirkstoffschicht (4, 14, 24, 34, 44, 65) höher als die Abgaberate der Basisschicht (12, 22, 32, 42, 62) ist.

12. Stent nach einem der Ansprüche 10 oder 11,
**dadurch gekennzeichnet,**
**dass** die Basisschicht (12, 22, 62) aus einem im menschlichen oder tierischen Körper nur langsam abbaubaren Polymer ausgebildet ist, wobei sich das Polymer über einen Zeitraum von 0,5 Monaten bis 24 Monaten, vorzugsweise 6 Monaten, abbaut.

13. Stent nach einem der Ansprüche 10 bis 12,
**dadurch gekennzeichnet,**
**dass** die Basisschicht (62) an unterschiedlichen Stellen eine unterschiedliche Schichtdicke aufweist, so dass die Basischicht (62) Täler (63) und Berge (64) ausbildet, wobei in den Tälern (63) die den Sofortwirkstoff (D2) haltende Wirkstoffschicht (65) vorgesehen ist.

14. Stent nach einem der Ansprüche 10 oder 11,
**dadurch gekennzeichnet,**
**dass** die Basisschicht (32, 42) als eine in die Oberfläche des Stents (30, 40) eingelassene Aussparung (3, 32, 42) ausgebildet ist.

15. Stent nach einem der Ansprüche 10 oder 11,
**dadurch gekennzeichnet,**
**dass** die Wirkstoffschicht (44) aus einem keramischen Material, insbesondere aus Aluminiumoxid, gebildet ist, welches durchgehende Poren oder Kanäle (49) aufweist, wobei in den Poren oder Kanälen (49) der Sofortwirkstoff (D2) eingelagert ist.

16. Stent nach Anspruch 15,
**dadurch gekennzeichnet,**
**dass** die aus einem keramischen Material, insbesondere aus Aluminiumoxid, gebildete Wirkstoffschicht (44) von einer Schutzschicht (48) aus einem nur langsam abbaubaren und wirkstoffdurchlässigem Polymer abgedeckt ist.

17. Stent nach einem der Ansprüche 10 bis 14,
**dadurch gekennzeichnet,**
**dass** die Wirkstoffschicht (65) ein Trägermaterial aus einem nur langsam abbaubaren und wirkstoffdurchlässigen Polymer aufweist, welches den Sofortwirkstoff (D2) hält.

18. Stent nach einem der Ansprüche 10 bis 12,
**dadurch gekennzeichnet,**
**dass** die Wirkstoffschicht (4, 14, 24, 34) ein sich im Körper abbauendes Trägermaterial aufweist, welches den Sofortwirkstoff (D2) hält.

19. Stent nach Anspruch 18,
dass dieses abbaubare Trägermaterial eine Emulsion oder ein schnell abbaubares Polymer ist.

20. Stent nach Anspruch 18,
dass die Emulsion eine Wasser in Öl -, Öl in Wasser - oder eine Lebertranemulsion ist.

21. Stent nach Anspruch 18,
**dadurch gekennzeichnet,**
**dass** das schnell abbaubare Polymer ein Polyphosphazen, ein Polyanhydrid, ein Polydioxan, ein Polyacetal, ein Polyoxalat, ein Polyester, PVP oder ein Derivat eines der vorgenannten Materialien ist.

22. Stent nach einem der Ansprüche 10 bis 21,
**dadurch gekennzeichnet,**
**dass** zwischen der Wirkstoffschicht (14) und der Basisschicht (12) oder zwischen der Wirkstoffschicht (34) und dem Stent (30) eine wirkstoffdurchlässige Schutzschicht (16, 36) aus einem nur langsam abbaubaren Polymer angeordnet ist.

23. Stent nach einem der Ansprüche 12, 14, 15 oder 22,
**dadurch gekennzeichnet,**
**dass** das nur langsam abbaubare Polymer aus einem Polymethylacrylat, aus PC, PA, PET, PE, PVA, PESU, PUR, PAN, PEO, Polyacryl, Polycarboxylsäure, ein Derivat eines der vorgenannten Materialien oder einer Kombination einiger der vorgenannten Materialien gebildet ist.

24. Stent zur Implantation in einen Lumen eines menschlichen oder tierischen Körpers, mit einem in einer Basisschicht (62) am Stent (60) lösbar gehaltenen Wirkstoff (D) und mit einem in einer zusätzlichen Wirkstoffschicht (65) in der weiterer Wirkstoff (D) gehalten ist, wobei der Wirkstoff (D) in der Basisschicht (62) über einen längeren Zeitraum an den Körper abgegeben wird, als der in der Wirkstoffschicht (65) gehaltene Wirkstoff (D),
**dadurch gekennzeichnet,**
**dass** die Basisschicht (62) an unterschiedlichen Stellen eine unterschiedliche Schichtdicke aufweist, so dass die Basischicht (62) Täler (63) und Berge (64) ausbildet, wobei in den Tälern (63) die den Sofortwirkstoff (D2) haltende Wirkstoffschicht (65) vorgesehen ist.
